# EUROPEAN PATENT SPECIFICATION

(11) **EP 3 291 723 B1**
(45) Date of publication and mention of the grant of the patent: **31.07.2024**
(21) Application number: 16726428.2
(22) Date of filing: 07.05.2016
(51) Int. Cl.: A61B 5/00, A61B 5/0215, A61B 17/00, A61B 17/12

(54) **HEART IMPLANT WITH SEPTUM GRIPPER**
HERZIMPLANTAT MIT SEPTUMGREIFER
IMPLANT CARDIAQUE À ORGANE DE PRÉHENSION DE SEPTUM

(30) Priority: 07.05.2015 US 201562157991 P; 10.03.2016 US 201662306134 P
(43) Date of publication of application: 14.03.2018
(73) Proprietor: Vectorious Medical Technologies Ltd., 6971921 Tel Aviv (IL)
(72) Inventor: GOLDSHTEIN, Oren, 22447 Nahariya (IL); LEVI, Yair, Haifa (IL); HARARI, Boaz, 55900 Ganey Tikva (IL)
(74) Representative: Beck Greener LLP
(86) International application number: PCT/IB2016/052616
(87) International publication number: WO 2016/178196

(56) References cited:
- WO-A1-2014/170771
- WO-A2-2008/042229
- US-A1- 2005 065 589
- US-A1- 2005 288 596
- US-A1- 2009 192 381
- US-A1- 2012 022 507

## Description

### FIELD OF THE INVENTION

The present invention, in some embodiments thereof, relates to implants, and more particularly, but not exclusively, to an implant, such as a sensory implant positionable within a left atrium in subject's heart, being configured and suitable for facilitating functional activity thereof. Such functional activity may include, for example, sensing, measuring, and, optionally, monitoring, physiological conditions or/and parameters in a subject's body organ.

### BACKGROUND OF THE INVENTION

Pressure measurements in one or more chambers of the heart may be used as an indicator for several cardiac conditions. By sensing and measuring cardiac pressure, abnormal events can be detected and appropriate treatment methodology may be applied to avoid otherwise deleterious cardiac conditions. For example, monitoring left atrial pressure of congestive heart failure (CHF) patients is commonly done as part of effectively diagnosing an abnormal change in left atrial pressure.

Prior art teaches about implants capable of sensing and measuring pressure in a heart atrium, and which can be fixed to the atrial wall (such as the atrial septum). Means are required for deploying and fixating such implants to the atrial wall. Typically, there is need to prevent, or at least diminish, potentially undesirable or problematic short-term or/and long-term effects that local or/and surrounding disturbances may impose upon the sensing and measuring, or/and other capabilities, of such heart atrial implants. There is also need for such sensory implants to be safely and effectively implanted within the heart, and usable for long-term, real time, and reliable cardiac pressure measurements in the right atrium or/and the left atrium.

PCT publication 2014/1700771 describes a remotely powered sensory implant having openings to minimize transmission distortions.

PCT publication 2008/042229 describes an implant catheter attachment mechanism.

US patent publication 2005/0288596 describes an implantable pressure transducer and systems for anchoring sensors to body structures.

### SUMMARY OF THE INVENTION

The present invention, in some embodiments thereof, relates to implants, and more particularly, but not exclusively, to an implant, such as a sensory implant positionable within a left atrium in subject's heart, being configured and suitable for fixation or/and stabilization by gripping on to both sides of a septal wall to thereby facilitating functional activity thereof. Such functional activity may include, for example, sensing or/and measuring, and, optionally, monitoring, physiological conditions or/and parameters in a subject's body organ.

According to an aspect of the present invention, there is provided an implant for sensing or/and measuring a physiological parameter in a subject's body organ separated by an organ wall as set out in the appending independent claim. Features of various embodiments are set out in the appending dependent claims.

There is also disclosed herein examples of an implant for sensing or/and measuring a physiological parameter in a subject's body organ separated by an organ wall, the implant includes an implant body having a body wall and enclosing an inner lumen sized and configured for housing physiological condition or/and parameter sensing or/and measuring means. In examples, such sensing or/and measuring means are miniature in size (so as to fit into, and be positionable inside of, a heart atrium), and include wireless communication means. In examples, the implant further includes a septum gripper having a flexible gripping sleeve selectively shapeable from a delivery mode narrowed form sized to pass through a septum opening in an atrial septum, to a deployment mode (expanded) septum gripping form.

Also disclosed are examples of a system for sensing or/and measuring a physiological parameter in a subject's body organ separated by an organ wall, the system including: the herein disclosed implant, a process control / data-information processing unit located outside of the subject's body and configured for interactively communicating with a physiological parameter sensing or/and measuring means of the implant; and a transcatheter implant deployment device configured for implanting or/and retrieving the implant.

Also disclosed are examples of an implant suitable for implantation in a subject's body organ separated by an organ wall, and configured for inductive coupling with an electronic unit located outside the subject's body and for interactively communicating with the electronic unit.

There is also disclosed herein examples of an implant for sensing or/and measuring a physiological parameter in a subject's body organ separated by an organ wall, the implant comprising: an implant body comprising a rigid body wall enclosing an inner lumen sized and configured for housing physiological parameter sensing or/and measuring means, said body wall includes coupling means at a proximal end thereof and configured for coupling with a distal end of an implant deployment device; and a septum gripper comprising a flexible gripping sleeve fixedly connected to periphery of said rigid body wall at a sleeve fixation area between center point and distal end of said body wall, said gripping sleeve is configured and selectively shapeable from a narrowed or/and stretched form, sized to pass through a septum opening in the organ wall, to a radially expanded form, suitable for gripping onto the organ wall from opposite sides thereof and around said septum opening, such that said implant body is fixatable or/and stabilizable across the organ wall and through said septum opening, so as to facilitate the sensing or/and measuring in the subject's body organ.

According to some examples, the subject's body organ is a left atrium in the subject's heart, and the organ wall is an interatrial septum.

According to some examples, the gripping sleeve is fixedly extendable or/and compressible to a chosen length or/and shape in between more than two distinct forms, including said narrowed form and said expanded form, when said coupling means are coupled with said distal end of said implant deployment device.

According to some examples, the septum gripper includes a mesh. According to some examples, the mesh is formed by braiding of at least one wire. According to some examples, the wire is made from metal alloy.

According to some examples, the gripping sleeve includes a bellows-like structure comprising a plurality of foldable units, including a proximal foldable unit and a distal foldable unit. According to some examples, when said gripping sleeve is in said expanded form, said proximal foldable unit forms a proximal wing extending outwardly-radially relative to said implant body, so as to form a first proximal surface and a second proximal surface interconnected with a proximal edge, and said distal foldable unit forms a distal wing extending outwardly-radially relative to said implant body, so as to form a first distal surface and a second distal surface interconnected with a distal edge.

According to some examples, the bellows-like structure is configured for gripping the organ wall, by pressing said second proximal surface or/and said proximal edge against a proximal side of the organ wall and pressing said second distal surface or/and said distal edge against a distal side of the organ wall, under a continuous compression force greater than a predetermined minimal force value. According to some examples, the bellows-like structure is configured with a coefficient of static friction chosen so as to prevent relative motion of said septum gripper relative to the organ wall under normal stresses applied thereto in the subject's body organ.

According to some examples, the proximal foldable unit and said distal foldable unit are spaced or/and interconnected with, and foldable relative to, a spacing unit. According to some examples, the spacing unit has a length equal to or smaller than a maximal thickness of the organ wall.

According to some examples, when said gripping sleeve is in said expanded form, said first proximal surface and said second proximal surface are formed as nested conic structures extending proximally away from said proximal edge, or formed as parallel flat structures, separated by said proximal edge; or/and said first distal surface and said second distal surface are formed as nested conic structures extending distally away from said distal edge, or formed as parallel flat structures, separated by said distal edge. According to some examples, the septum gripper, when gripping onto the organ wall, is configured to have a chosen tridimensional orientation relative to a center point of said septum opening. According to some examples, the chosen orientation includes said implant body extending through said septum opening such that an operational member of said implant body is positionable inside of the subject's body organ. According to some examples, the operational member includes a pressure sensor configured for the sensing or/and measuring when positioned within the body organ remotely from the organ wall.

According to some examples, the septum gripper comprises a non- foldable sleeve portion, wherein said septum gripper sleeve is connected to said body wall only via said non-foldable sleeve portion. According to some examples, the non- foldable sleeve portion is sandwiched between interconnecting sleeve fixation inner ring and sleeve fixation outer ring affixed to said sleeve fixation area. According to some examples, the sleeve fixation inner ring is sized and shaped to mate or/and lock in a recess forming said sleeve fixation area, to thereby prevent axial and rotation movement therebetween. According to some examples, the non-foldable sleeve portion has an inner dimension of about 2.5 mm or less. According to some examples, the non- foldable sleeve portion is provided between said proximal foldable unit and said distal foldable unit. According to some examples, the non-foldable sleeve portion is provided proximally to said proximal foldable unit. According to some examples, the non-foldable sleeve portion is provided distally to said distal foldable unit.

According to some examples, at least one of said proximal edge and said distal edge has an outer dimension of about 2.5 mm or more. According to some examples, the gripping sleeve has super elastic properties, or/and shape memory properties. According to some examples, the gripping sleeve is formed by pressing over a rigid mandrel shaped and dimensioned with inner dimensions of said gripping sleeve in said expanded form. According to some examples, the gripping sleeve is encapsulated with an electrically non-conductive layer. According to some examples, the electrically non-conductive layer separates or/and spaces between said gripping sleeve and electrically conductive portions of, or connected to, said body wall.

According to some examples, the septum gripper includes or is connected to a sliding element configured for unhindered sliding along said body wall during shifting between said narrowed form and said expanded form.

According to some examples, wherein a free end of said gripping sleeve is sandwiched between interconnecting sleeve deploying inner ring and sleeve deploying outer ring, wherein said sleeve deploying inner ring comprises said sliding element and said sleeve deploying outer ring is configured for coupling with a sleeve deploying arm provided with said implant deployment device.

According to some examples, the body wall includes a first elongated wall segment of a first outer dimension and a second elongated wall segment of a second outer dimension smaller than said first outer dimension, wherein said gripping sleeve is configured to cover said first and second wall segments when in said narrowed form and to expose said first wall segment when in said expanded form. According to some examples, the second wall segment houses a metal coil or/and an antenna configured for transmitting or/and receiving radio-frequency electromagnetic waves. According to some examples, the first wall segment is formed of metal alloy, and said second wall segment is formed of ceramic material. According to some examples, the implant further comprises a third wall segment distally to said second wall segment, configured for housing electronic or/and electro-mechanical components. According to some examples, the third wall segment is formed of metal alloy.

There is also disclosed herein examples of a system for sensing or/and measuring a physiological parameter in a subject's body organ separated by an organ wall, the system comprising: an implant comprised of: an implant body comprising a rigid body wall enclosing an inner lumen sized and configured for housing physiological parameter sensing or/and measuring means, said rigid body wall includes coupling means at a proximal end thereof and configured for coupling with a distal end of an implant deployment device; and a septum gripper comprising a flexible gripping sleeve fixedly connected to periphery of said rigid body wall at a sleeve fixation area between center point and distal end of said body wall, said gripping sleeve is configured and selectively shapeable from a narrowed or/and stretched form, sized to pass through a septum opening in the organ wall, to a radially expanded form, suitable for gripping onto the organ wall from opposite sides thereof and around said septum opening, such that said implant body is fixatable or/and stabilizable across the organ wall through said septum opening, so as to facilitate the sensing or/and measuring in the subject's body organ; a process control / data-information processing unit, located outside of the subject's body, and configured for interactively communicating with said physiological parameter sensing or/and measuring means of said implant; and a transcatheter implant deployment device configured for implanting or/and retrieving said implant.

There is also disclosed herein examples of an implant suitable for implantation in a subject's body organ separated by an organ wall, and configured for inductive coupling with an electronic unit located outside the subject's body and for interactively communicating with the electronic unit, the implant comprising: an implant body comprising a rigid body wall having an electrically non-conductive wall section, housing an RF antenna configured for facilitating the inductive coupling or/and the interactive communication with the electronic unit; and a septum gripper comprising: an electrically conductive flexible gripping sleeve provided between said non-conductive wall section and the electronic unit, and encompassing said non-conductive wall section; an electrically non- conductive distal inner ring fixedly connecting distal portion of said gripping sleeve to said implant body; and an electrically non-conductive proximal inner ring connecting proximal portion of said gripping sleeve to said implant body and allowing sliding thereof lengthwise about outer periphery of said rigid body wall between a proximal-most location and a distal- most location; wherein said gripping sleeve is configured and selectively shapeable from a narrowed or/and stretched form when said proximal inner ring is positioned at said proximal- most location, to an expanded form when said proximal inner ring is positioned at said distal- most location.

According to some examples, the flexible gripping sleeve includes an electrically conductive mesh. According to some examples, the gripping sleeve includes a bellows-like structure comprising a proximal foldable unit and a distal foldable unit, wherein, when said gripping sleeve is in said expanded form, said proximal foldable unit forms a proximal wing extending outwardly-radially relative to said implant body, so as to form a first proximal surface and a second proximal surface interconnected with a proximal edge, and said distal foldable unit forms a distal wing extending outwardly-radially relative to said implant body, so as to form a first distal surface and a second distal surface interconnected with a distal edge. According to some examples, the distal inner ring is fixated to said rigid body wall at a sleeve fixation area between center point and distal end of said body wall.

According to some examples, the gripping sleeve, when in said narrowed or/and stretched form, is sized to pass through a septum opening in the organ wall. According to some examples, the gripping sleeve, when in said radially expanded form, is configured for gripping onto the organ wall from opposite sides thereof and around said septum opening, such that said implant body is fixatable or/and stabilizable across the organ wall through said septum opening, so as to facilitate the inductive coupling or/and the interactive communication with the electronic unit.

According to some examples, the septum gripper further comprises a distal outer ring sandwiching said distal portion of said gripping sleeve with and around said distal inner ring, and a proximal outer ring sandwiching said proximal portion of said gripping sleeve with and around said proximal inner ring. According to some examples, the septum gripper includes a layer of electrically non-conductive covering material configured for separating or/and spacing between electrically conductive parts thereof and said body wall. According to some examples, the gripping sleeve is encapsulated with said electrically non-conductive covering material. According to some examples, the distal outer ring or/and said proximal outer ring is electrically conductive.

According to some examples, the rigid implant body has a total length in a range of between about 5 mm and about 50 mm, optionally, between about 10 mm and about 30 mm, optionally, between about 15 mm and about 20 mm, optionally, about 18 mm, and has a maximal outer diameter being about 5 mm or less, optionally, about 3.5mm or less, or optionally, about 2 mm or less.

According to some examples, the tubular skirt in its extended and narrowed form has a length in a range of between about 25 mm and about 100 mm, optionally, about 50 mm in length, and is sized for unhindered passage when constricted to a diameter in a range from about 2 mm to about 4 mm, such as through a 12 French outer sheath / catheter (i.e., about 4 mm outer diameter) or/and is about 6 mm or less in diameter if not constricted. The septum opening is optionally substantially smaller than the outer sheath outer diameter but is elastically stretched wide when passing therethrough, optionally, about 1 mm to about 3 mm (slit or puncture), optionally using a 5 to 8 French (1.25 mm to 2.67 mm) trans-septal puncture kit. Tubular skirt may be loaded into the outer sheath via a gradually constricting passage, such as from about 7-10 mm down to about 3.8-4.2 mm in diameter.

When in the (radially expanded) gripping form, the tubular skirt may be less than about 25 mm, optionally, about 15mm or less in length, and about 10 mm or more, optionally, about 15 mm or about 18 mm or more, in diameter. The tubular skirt may be formed of a mesh having intertwined members (e.g., fibers, cords, or the like), optionally, made of metal (e.g., Ni-Ti alloy). In examples, the mesh includes 42 intertwined (one over one) Ni-Ti 100 micron fibers/cords fiber diameter, optionally, with light oxide surface finish, and 90-degree intersection angle.

There is also disclosed herein examples of a wireless pressure sensor implant, provided in a miniature packaging, with a dedicated delivery system. According to some examples, the implant is a part of a sensory system (also comprising a remote device applicable from outside subject's body and configured for monitoring physiological data from within the human body, for example, direct pressure measurement from within the heart's left atrium.

According to some examples, the implant includes anchoring means designed for being fixated on to a wall of an internal body organ, for example, the inter-atrial septal wall, and implantable via a minimal invasive procedure.

According to some examples, the implant includes sensing or/and measuring elements integrated into a tube (tubular) shaped (rigid) implant body which houses electronic circuitry.

According to some examples, the implant includes several sub-assemblies, for example: (i) a sensory element cup assembly that includes electrical feedthrough, which can house, for example, one or more pressure sensing or/and measuring transducers positionable on one or both ends of the implant, (ii) a brazed tube assembly including a tube applied with a braided (mesh) fixation means, and (iii) an inner electronic assembly that includes electronic circuitry for interfacing with the sensing or/and measuring element and communicating the data to the external unit.

According to some examples, the inner electronic assembly may include one or more of the followings: (i) a dedicated application- specific integrated circuit (ASIC) that is mounted on a miniature printed circuit board (PCB) that is applied with other discreet elements, (ii) an RF antenna, which is electrically connected to the PCB and includes a metallic core wrapped with a conductive coil.

The implant body (tube) may be about 1 mm, 2 mm, 2.5 mm, 3.5 mm, or up to about 5mm, in outer diameter, and about 8 mm, 10 mm, or up to about 22 mm, in length. The fixation means (e.g., a septum gripper, optionally, including a mesh or a braided pattern) may be from about 10 mm to about 24 mm wide / diameter, when fully deployed (radially expanded). These fixation means, when crimped (e.g., stretched or/and narrowed) are optionally deliverable through the lumen of an 8-French (Fr), 10-Fr, 11-Fr, 12-Fr, or a larger diameter, or a smaller diameter, sheath.

According to some examples, the implant body is made of a metal with possible combination with ceramic, for example, in the form of a metal-ceramic composite or composite type material. The implant body is optionally configured particularly to endure internal body environment providing biocompatibility and structural strength, and to allow sufficient radio frequency (RF) interactive communication between the implant and its external unit for transmitting / receiving power and digital information, and, to process and transmit / receive physiological parameters and information from the sensor. Optionally, implant body segment which houses the antenna may be an RF compatible tube, and may be either non- metallic / electrical non-conductive (optionally, made from zirconia or titania) or metallic with grooves. An exemplary non-metallic tube is ceramic.

According to some examples, the implant fixation means (septum gripper) is made of either a metallic and/or polymeric material or with any other material combination that is either electrical conductive or electrical non-conductive in nature. Optionally, the fixation means include a mesh (e.g., braid) made from Ni-Ti alloy ('Nitinol'), which is thermally shaped to preformed double- sided wall gripping form shape, and is configured for mechanical stabilization in human body temperature, yet deformable for allowing forming from a crimped (narrowed or/and stretched) form to its expanded form.

According to some examples, the implant fixation means (septum gripper) is connected to the rigid implant body using several designated fixtures. Optionally, at least one of the fixtures includes inner and outer rings configuration, sandwiching a portion of the septum gripper (sleeve / mesh) therebetween. Fixtures may optionally be made from electrically non-conductive materials for isolating the septum gripper (sleeve) from electrically conductive parts of the implant body, as well as for sealing and mechanically protecting outer edges of the sleeve (mesh / braid). Two fixtures may exist for both the proximal end and the distal braid end.

According to some examples, the implant is deliverable, deployable, or/and fixatable, using a delivery kit or system which may include a trans-septal outer sheath and dilator kit, an implant loader (into outer sheath), and an implant delivery/deployment device which may include a rod-like element or a cable connectable to the implant and applicable for pushing the implant through the outer sheath until reaching the target deployment location, and be released after the implant has been positioned properly. Prior to implantation with the dedicated delivery system, the septal wall may be punctured using a standard trans-septal puncturing kit in accordance to known practice.

According to some examples, the implant is positioned across the interatrial septum. Optionally, implantation is carried out following standard trans-septal puncture and passage typically using a femoral venous approach. Following sheath and wire positioning in the left atrium, or alternatively, in a pulmonary vein, the implant may be introduced through the sheath to a chosen position or/and orientation in the left atrium. The implant is optionally inserted either preloaded within a dedicated catheter or pushed directly through the outer sheath prior to deployment and fixation across the interatrial septum.

According to some examples, the implant is first introduced partially within the left atrium followed by deployment (radial expansion) of gripping sleeve distal end only, and it is slowly and safely exposed within the left atrium by gradual retraction of the outer sheath. The septum gripper is shaped and configured for recollapsing into the outer sheath and allows for adjustment of the space between its gripping surfaces in conformity to wall anatomy (such as the interatrial septum thickness. Deployment of the proximal part of the septum gripper is optionally performed after fully exposing the septum gripper from the outer sheath, optionally under vision using fluoroscopy or/ and echocardiography. Following confirmation of device position across the interatrial septum and its stability, the implant can be detached from the implant deployment device. Complete retrieval of the implant is possible during the deployment at least until complete detachment of the deployment device from the implant.

According to some examples, the implant deployment procedure may include at least one of the following steps (not necessarily in same order):
> holding the outer sheath in place and slowly advancing the implant forward using the implant deployment device until the implant is aligned with the distal tip of the outer sheath.
> under echocardiography guidance or/and fluoroscopy, repositioning the outer sheath so that its distal tip is approximately 2-3 cm distal to the Fossa Ovalis inside the left atrium.
> retracting the outer sheath while keeping the implant in-place using the implant deployment device, until a chosen distal portion of the septum gripper (gripping sleeve) is exposed in the left atrium and radially expands.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

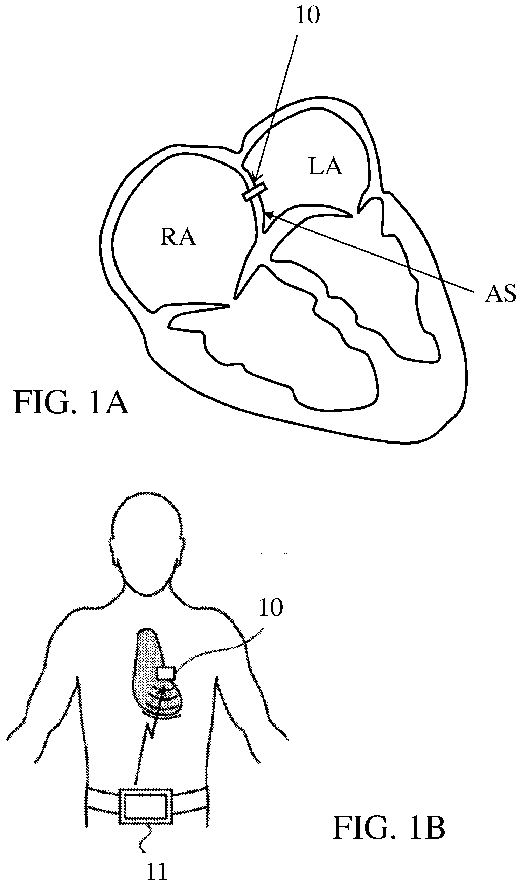

FIGs. 1A - 1B are a schematic views of a human heart (FIG. 1A), showing an exemplary implant configured for implantation in the atrial septum (AS) and to extend from the right atria (RA) to the left atria (LA), and communication capabilities between the implant and an external (electronic) unit (FIG. 1B);

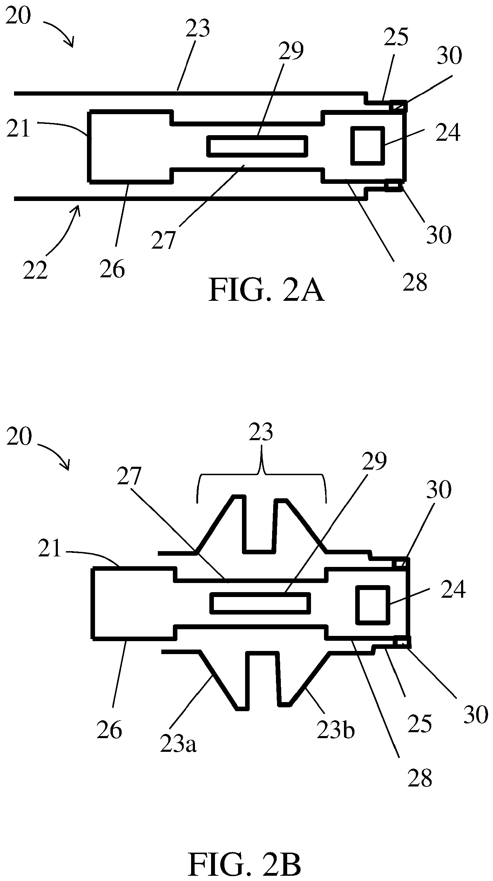

FIGs. 2 A - 2B are schematic side cut views of an exemplary implant, having an implant body, and a septum gripper that includes a proximal foldable unit and a distal foldable unit, the septum gripper is connected and fixated to the implant body at a sleeve fixation area located distally to the distal foldable unit;

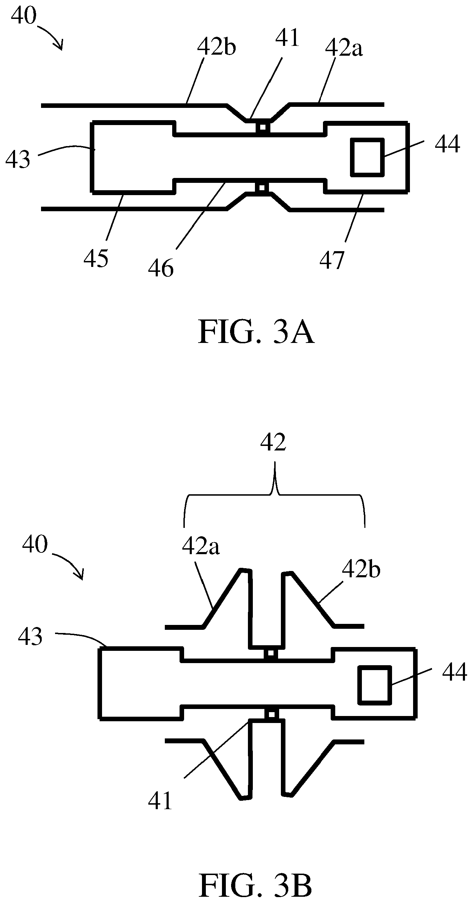

FIGs. 3A - 3B are schematic side cut views of an exemplary implant, having an implant body, and a septum gripper that includes a proximal foldable unit and a distal foldable unit, the septum gripper is connected and fixated to the implant body through sleeve fixation area being positioned in between the proximal and the distal foldable units;

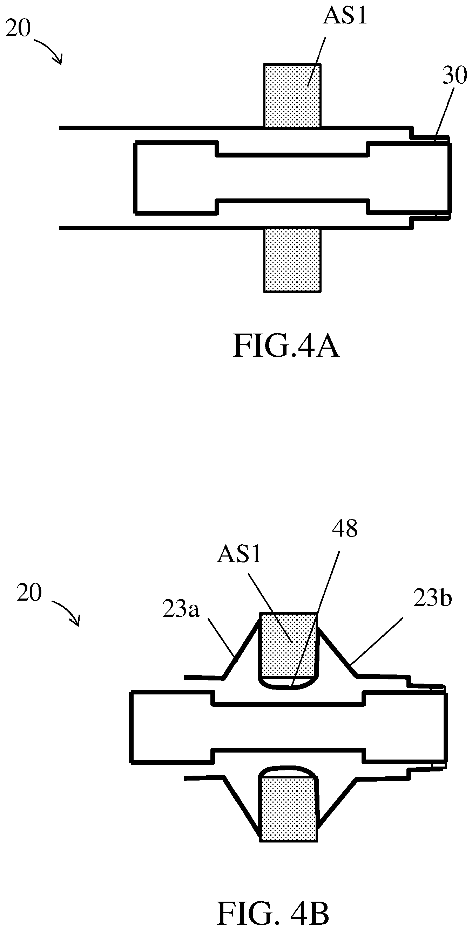

FIGs. 4A - 4B are schematic side cut views of an exemplary implant, when in the (delivery) narrowed or/and stretched form (FIG. 4A) configured to cross an atrial septum AS 1 having a first wall thickness, and the (gripping) radially expanded form (FIG. 4B) that grips the atrial septum AS 1 having a first wall thickness;

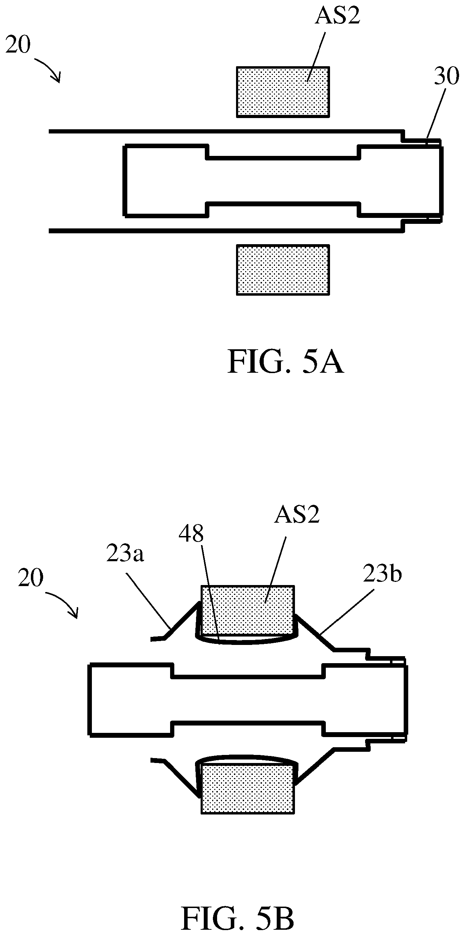

FIGs. 5A - 5B are schematic side cut views of an exemplary implant, when in the (delivery) narrowed or/and stretched form (FIG. 5A) configured to cross an atrial septum having a second wall thickness AS2, and the (gripping) radially expanded form (FIG. 5B) that grips the atrial septum AS2 having a second wall thickness;

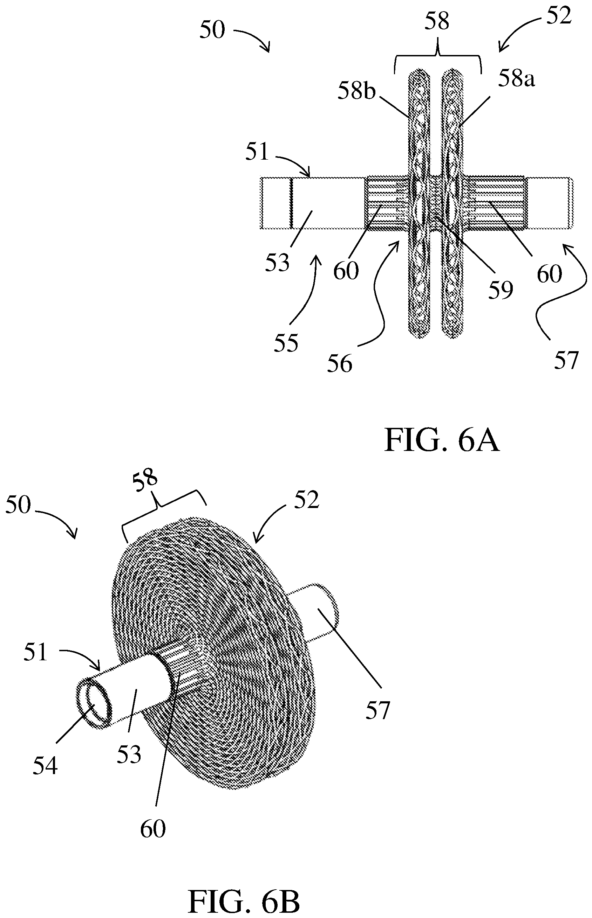

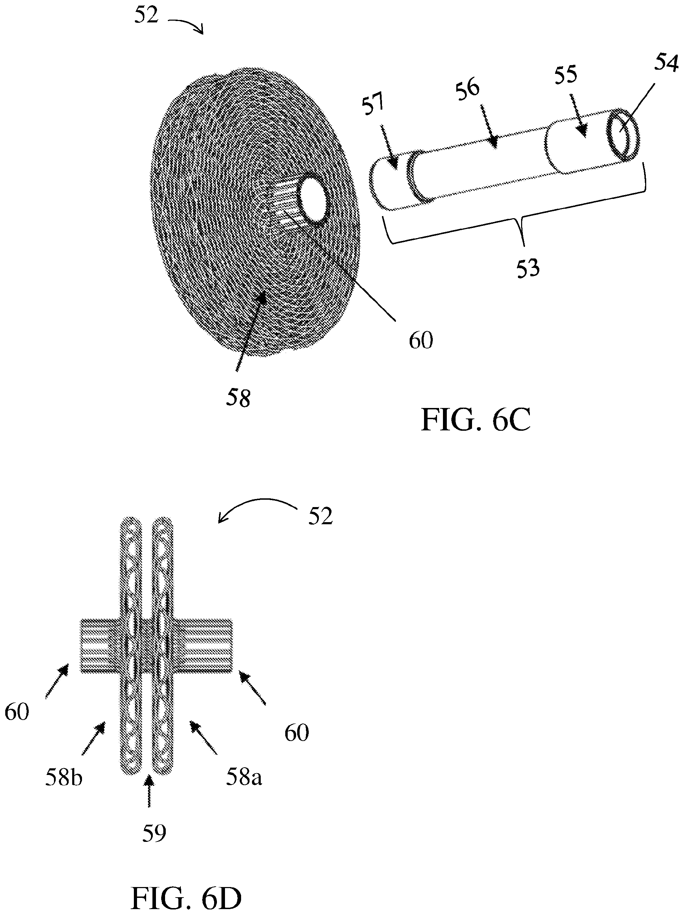

FIGs. 6A - 6D are side or isometric views of an exemplary implant in a (gripping) radially expanded form, the implant is having an implant body and a griping sleeve including a mesh and suitable for continuous gripping an atrial septum;

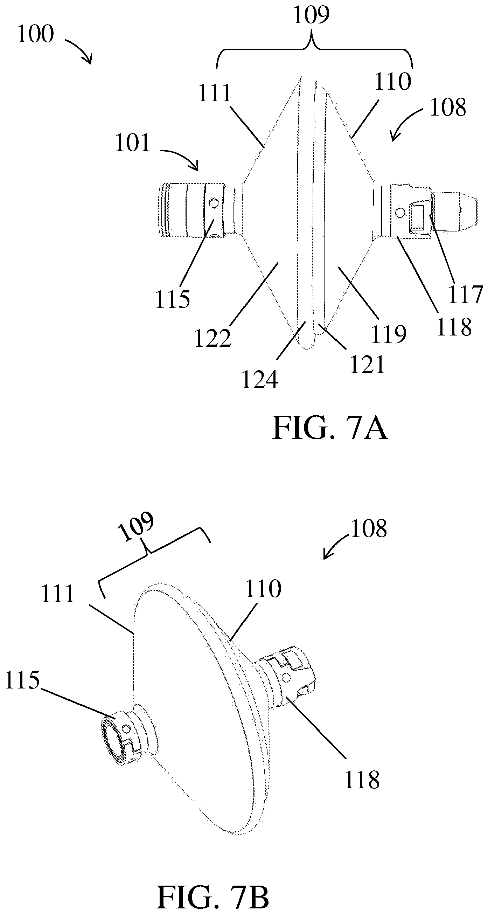

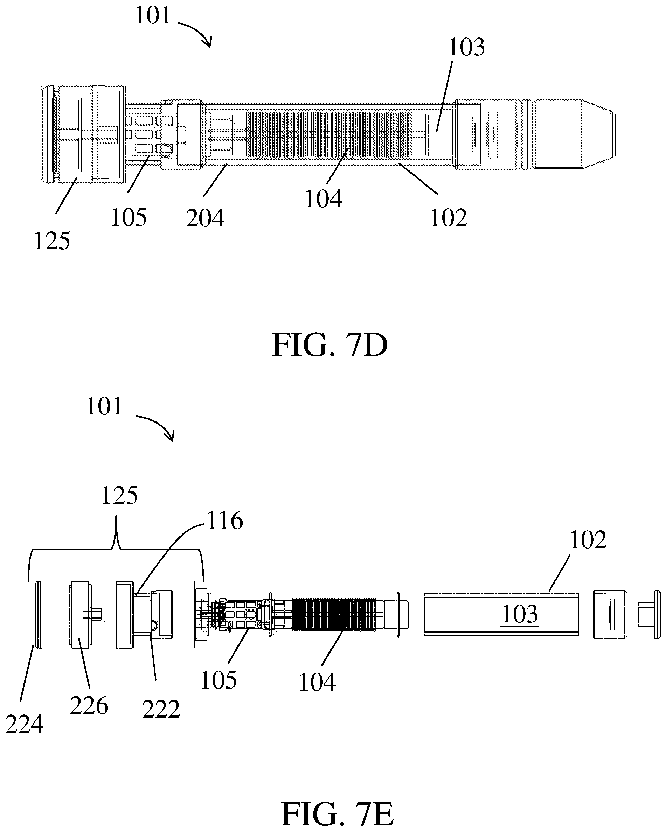

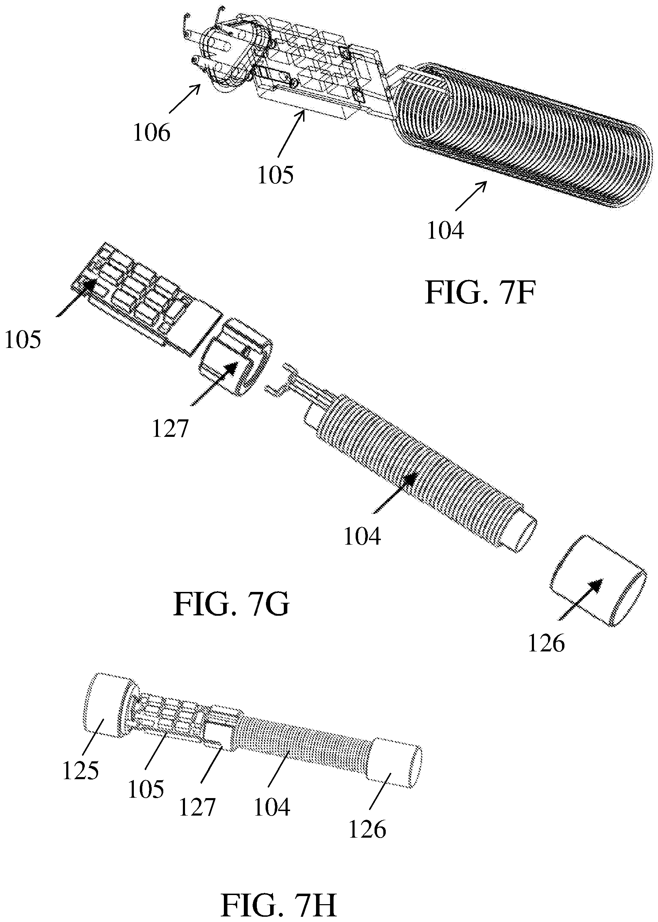

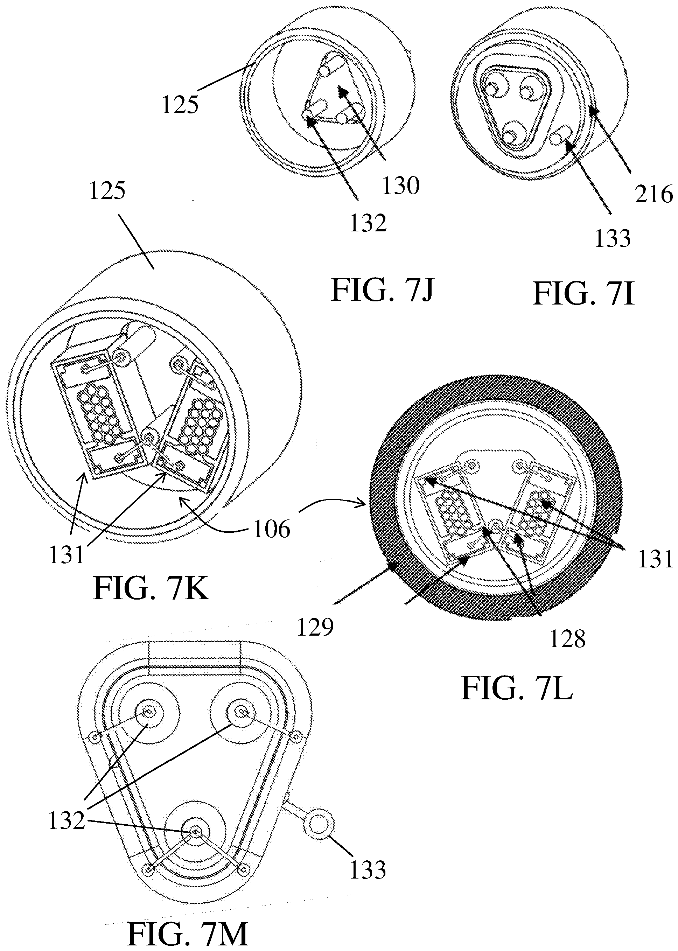

FIGs. 7A - 7M are side or isometric views of an exemplary implant, the implant is having an implant body and a griping sleeve that forms a bellows-like structure when in the gripping form (FIGs. 7A - 7C), the implant body is further having internal electronic components (FIGs. 7D - 7M); and

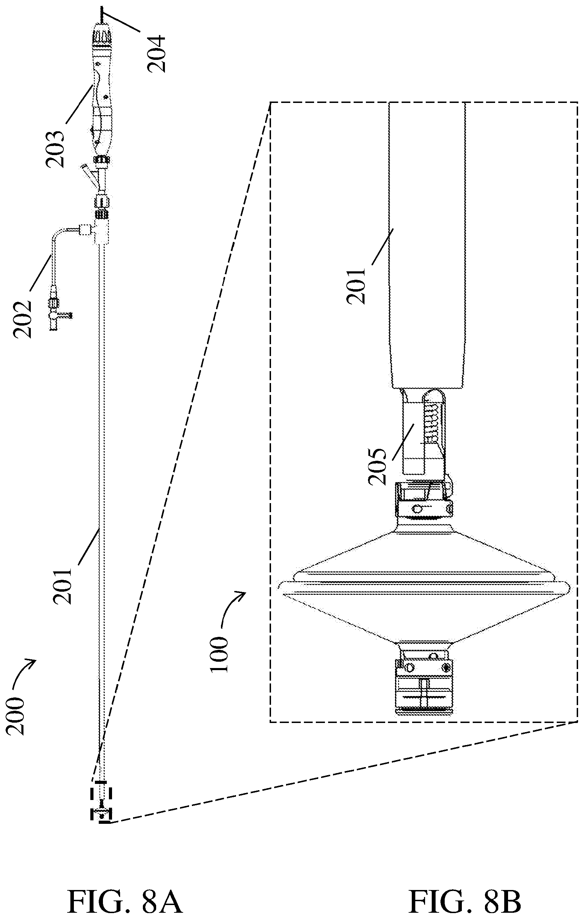

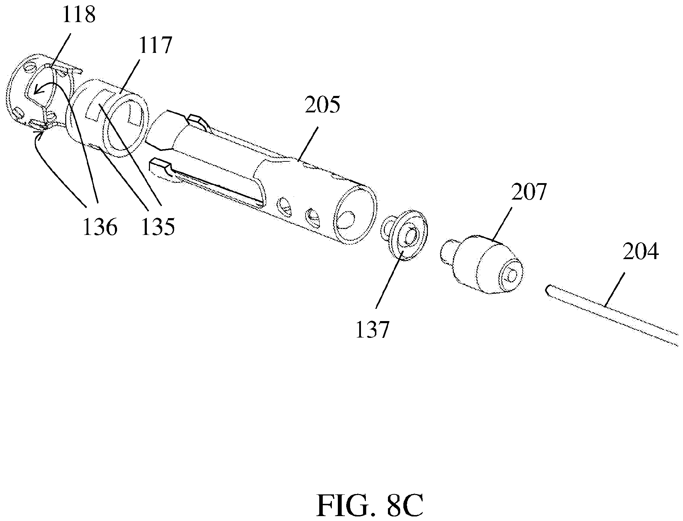

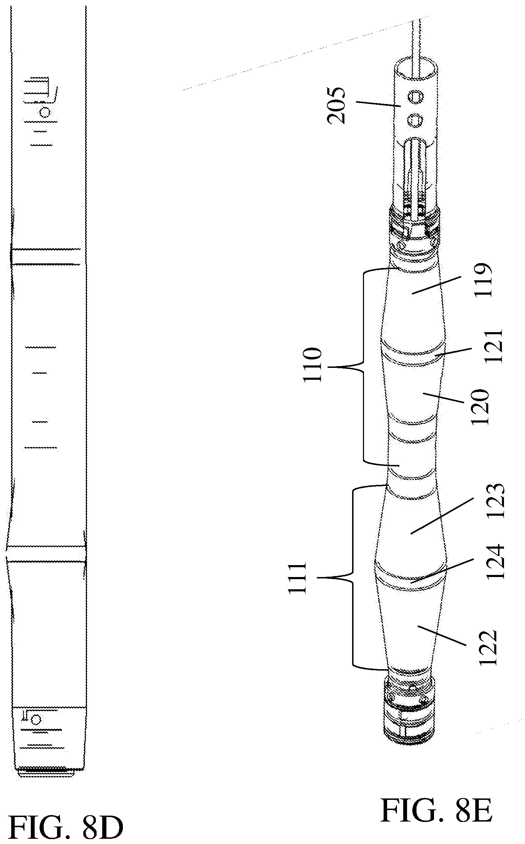

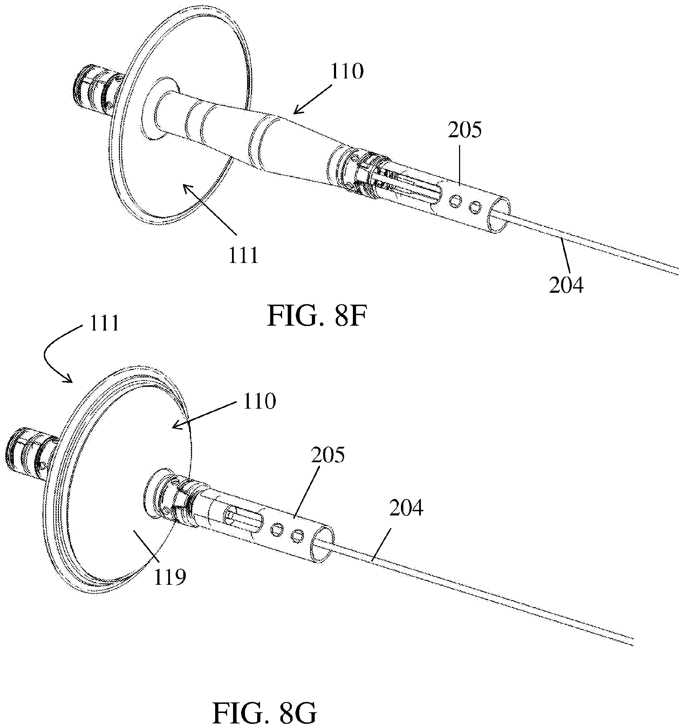

FIGs. 8A - 8G are schematic side or isometric views of an exemplary implant deployment device configured for implantation and deployment of the herein disclosed exemplary implants.

### DESCRIPTION OF SPECIFIC EMBODIMENTS OF THE INVENTION

The present invention, in some embodiments thereof, relates to implants, and more particularly, but not exclusively, to an implant, such as a sensory implant positionable within a left atrium in subject's heart, being configured and suitable for fixation or/and stabilization by gripping on to both sides of a septal wall to thereby facilitating functional activity thereof. Such functional activity may include, for example, sensing or/and measuring, and, optionally, monitoring, physiological conditions or/and parameters in a subject's body organ.

In the field and art of the invention, there is a requirement to provide wall mounting / fixating means for implants, particularly implants which are deliverable (e.g., transcatheter delivery) into a body organ that is separated by an organ wall, where the implant is to reside across this organ wall. Usually, such an implant is provided across a naturally occurring opening (e.g., a normal opening or an abnormal / defect opening) or a premade opening (e.g., hole, cut, or puncture) and is fixated by pressing against the two sides of the organ wall around the wall opening. The inventors of the present invention observed that there is still an on-going need for such fixating / mounting means in view of the following conditions or/and requirements, singly, or in combination:
> such means should be stored and delivered in a very small diameter constrictive channels, for example, diameters of 4 mm, or even 3 mm and less, without deteriorating their structure and function when expanded for fixating the implant to the organ wall.
> such means should be able to inherently compensate for different dimensions of the wall, for example wall thickness which may be substantially variable, without compensating for their structure and function when expanded for fixating the implant to the organ wall.
> such means should be able to deform into final fixation (e.g., gripping) form without relying on anatomy, as in current practice whereby deployment of trans-septal implants (e.g., septal occluders) is done by extracting the implant (thereby allowing its fixation means to self-expand) out of an outer sheath against the organ wall margins surrounding the wall opening, yet the opening size may not allow that practically or efficiently.
> such means should be able to recollapse and retrieve for optional discarding or redeployment (e.g., in a different target location), since that, especially with respect to sensory implants, implant's functionality can be determined only after deployment.

In exemplary embodiments, the implant includes an implant body having a body wall and enclosing an inner lumen sized and configured for housing physiological condition or/and parameter sensing or/and measuring means. In exemplary embodiments, such sensing or/and measuring means are miniature in size (so as to fit into, and be positionable inside of, a heart atrium), and include wireless communication means. In exemplary embodiments, the implant further includes a septum gripper having a flexible gripping sleeve selectively shapeable from a delivery mode narrowed form sized to pass through a septum opening in an atrial septum, to a deployment mode (expanded) septum gripping form.

Also disclosed are exemplary embodiments of a system for sensing or/and measuring a physiological parameter in a subject's body organ separated by an organ wall, the system including: the herein disclosed implant, a process control / data-information processing unit located outside of the subject's body and configured for interactively communicating with a physiological parameter sensing or/and measuring means of the implant; and a transcatheter implant deployment device configured for implanting or/and retrieving the implant.

Also disclosed are exemplary embodiments of an implant suitable for implantation in a subject's body organ separated by an organ wall, and configured for inductive coupling with an electronic unit located outside the subject's body and for interactively communicating with the electronic unit.

Sensing or/and measuring, and optionally, monitoring, physiological conditions or/and parameters (such as pressure) within the left atrium or/and right atrium of the heart may be associated with several challenges and limitations. For example, access to the left or right heart atrium needs to be done in a safe manner. In addition, the one or more physiological sensors (sensing or/and measuring means) need to be implanted in a manner that ensures accurate sensing or/and measuring, and optionally, monitoring, of the physiological conditions or/and parameters (such as pressure) for a prolonged period of time (e.g., 3 years or more, optionally, particularly 5 years or more, or, optionally, particularly 10 years or more). At least some exemplary embodiments of the implant may successfully address and overcome such potential challenges and limitations, and may provide for safe, reliable, and accurate sensing or/and measuring, and optionally, monitoring, of one or more physiological conditions or/and parameters (such as pressure), within the left or/and right heart atrium.

In exemplary embodiments, the herein disclosed implant is implanted through the septum using minimal procedure (minimally invasive) means. In exemplary embodiments, the herein disclosed implant may be easily retrieved, and either replaced or completely removed, from within the heart, when and if necessary, at least so long as the implant is connectable to dedicated delivery / retrieval means. Such exemplary embodiments of the herein disclosed heart atrial septum implant may have structural and functional features that are advantageous compared to those of known heart atrial implants and methods used for employing them.

The implant affords measuring pressure within at least one of the left and right atrium, optionally particularly in the left atrium. Such measurements are of particular importance especially when referring to heart failure patients (e.g., CHF patients). In such modalities, continuous heart pressure monitoring may be effective for assessing and managing disease progression. Timely interventions including medication taken as immediately as possible after an increase in pressure would be considered more effective for applying timely treatment to a CHF patient or/and for reducing unnecessary hospitalization.

The implant thus fulfills a long felt need in providing heart pressure monitoring for long periods (e.g., about 5 years or more, optionally about 10 years or more).

Thus, the present invention provides an implant that includes sensing or/and measuring means extendable within a left atrium in a patient's heart, and fixatable (e.g., by gripping) to the heart atrial septum. Referring now to the drawings, FIG. 1A is a schematic frontal cut view of a human heart showing the atrial septum (AS). Atrial septum is the wall that separates the right atrium (RA) from the left atrium (LA) of the heart. This term is interchangeable with the term "interatrial septum". As shown in FIG. 1A and will be explained in more details below, the implant of the invention, is configured to be placed through or reside within the atrial septum. In FIG. 1A, a simplified illustration of implant 10 is shown.

The implant may include sensing or/and measuring means. The term "sensing or/and measuring means", as used herein, refers to component(s) or/and electronics that enable and facilitate sensing or/and measuring and transmitting data associated with one or more physiological conditions or/and parameters of the heart / cardiac system that may be processed to provide information regarding condition of a patient's heart. According to some embodiments, the mean(s) include at least a pressure sensor or transducer capable of specifically sensing or/and measuring, or measurably reacting to, pressure (or change thereof, or difference thereof with ambient pressure, for example) within the heart (e.g., particularly in the left atrium).

The term "sensing or/and measuring means", as used herein, is interchangeable with the term "operational member" or may be considered a component or a part thereof. The data transmitted from the sensing or/and measuring means may include, without limitation, at least one of the following physiological conditions or/and parameters: pressure, stress, heart rate, anatomical abnormalities (e.g., valve malfunction), temperature, blood flow rate, and gas (e.g., oxygen) concentration or/and volume. The data transmitted from the sensing or/and measuring means is transmitted wirelessly to an external unit 11 located outside patient's body (As shown in FIG. 1B), and configured for communicating (i.e., reading, processing and providing an input) with the sensing or/and measuring means, which includes for example reading, analyzing or/and processing a data (picked-up by the sensing or/and measuring means or/and combined with stored data or/and newly introduced data) to provide a readout by the external unit. According to some embodiments, the external unit may also provide power to the sensing or/and measuring means or/and to other components thereinside.

The implant includes a (selectively deployable) septum gripper configured to grip on to the septum, such as from both sides thereof. The septum gripper includes a first and a second foldable unit.

In exemplary embodiments, the first and the second foldable units are configured to shift from a delivery mode extended form to a post-delivery mode gripping form, such that, in the delivery mode form, the foldable units are extended and the implant is capable of passing through an opening of the septum, and in the post-delivery mode gripping form, the foldable units are deployed and the septum is gripped or clamped in-between the two foldable units.

The implant further includes at least one sensing or/and measuring means confined within the implant body and adapted to monitor physiological condition(s) or/and parameter(s) within a heart cavity.

The description that follows illustrates possible example embodiments of the implant and its delivery and anchoring (e.g., fixating, such as by gripping). The embodiments described herein are depicted purely by way of example, and various other suitable implementations can be used in alternative embodiments.

Reference is now made to FIGs. 2A - 2B, which schematically illustrate side cut views of an implant 20, in accordance with some embodiments of the invention. Implant 20 includes implant body 21 which houses sensing or/and measuring means 24 capable of sensing (detecting) or/and measuring, and optionally, monitoring, one or more physiological conditions or/and parameters in a chamber of the heart. Implant 20 further includes septum gripper 22 including gripping sleeve 23 shapeable from a narrowed (delivery) form (FIG. 2A) sized to pass through a septum opening in an atrial septum, to an expanded (gripping) form configured for gripping onto atrial septum (FIG. 2B). Gripping sleeve 23 includes proximal foldable unit 23a and distal foldable unit 23b (shown in FIG. 2B). Gripping sleeve 23 further includes a non-foldable sleeve portion 25 whose dimensions are constant during sleeve change in shape when the gripping sleeve 23 transforms from the narrowed form to the expanded form.

Implant body 21 of implant 20 includes a first elongated wall segment 26 of a first outer dimension and a second elongated wall segment 27 of a second outer dimension. Implant body 21 of implant 20 may further include a third elongated wall segment 28. According to some embodiments, the outer dimension of the second elongated wall segment 27 is smaller than the outer dimension of the first elongated wall segment 26.

According to some embodiments, first wall segment 26 and optionally, second elongated wall segment 27 is formed of metal alloy. Suitable metal alloys include, but are not limited to alloys including titanium (Ti). The Alloys may have a certain purity. For example, the Ti alloy may be of grade II. According to some embodiments, the second wall segment 27 is formed from a ceramic material such as zirconia or alumina. According to some embodiments, the second wall segment 27 houses a metal coil or/and an antenna 29 configured for transmitting or/and receiving radio-frequency electromagnetic waves. According to some embodiments, gripping sleeve 23 is connected to implant body 21 only through non-foldable sleeve portion 25. According to some embodiments, non-foldable sleeve portion 25 is connected to implant body 21 through sleeve fixation area 30. According to some embodiments and as shown in the FIG. sleeve fixation area 30 may be disposed distally to the distal foldable unit 23b.

The sleeve fixation area 30 may include an inner and an outer ring, wherein the outer ring clamps the inner ring which in turn clamps the distal non-foldable sleeve portion, thereby fixating distal non-foldable sleeve portion to implant body 21.

Reference is now made to FIGs. 3A - 3B, which show a schematic side cut views of implant 40.

Implant 40 includes a gripping sleeve 42 shapeable from a narrowed or/and stretched (delivery) form (FIG. 3A) sized to pass through a septum opening in an atrial septum, to a radially expanded (gripping) form configured for gripping onto atrial septum (FIG. 3B). Implant 40 is similar to implant 20, except for its sleeve fixation area 41 (which includes a non-foldable sleeve portion) being positioned in between proximal foldable unit 42a and distal foldable unit 42b of gripping sleeve 42. Similar to implant 20, implant 40 includes an implant body 43 which houses sensing or/and measuring means 44 capable of sensing (detecting) or/and measuring, and optionally, monitoring, one or more physiological conditions or/and parameters in a chamber of the heart. Implant body 43 of implant 40 includes a first elongated wall segment 45 of a first outer dimension a second elongated wall segment 46 of a second outer dimension and optionally a third elongated wall segment 47.

Reference is now made to FIGs. 4A - 4B, which show implant 20 when extending across a pre-made opening in a first atrial septum AS1, when in the delivery form (FIG. 4A), and in a gripping form (FIG. 4B), where it grips the atrial septum AS1. In this example the atrial septum (wall) is relatively thin.

FIGs. 5A - 5B show implant 20 first extending across a pre-made opening in a second, relatively thick, atrial septum (wall) AS2, when in the delivery form (FIG. 5A), and then in a gripping form (FIG. 5B), where it grips the atrial septum AS2. Atrial septum wall thickness may vary substantially between patients. Typically, atrial septum wall-thickness ranges between about 1 mm to about 20 mm. Implant 20 is configured to accommodate various atrial septum wall thicknesses and is therefore adjustable to all atrial septum wall sizes. Specifically, at least one of: proximal foldable unit 23a, spacing unit 48, and distal foldable unit 23b are elastically stretchable and at the same time maintain septum gripping functionality.

Reference is now made to FIGs. 6A - 6D which show implant 50.

Implant 50 includes an implant body 51 configured for housing means capable of sensing (detecting) or/and measuring, and optionally, monitoring, one or more physiological conditions or/and parameters in a chamber of the heart (hereinafter "sensing or/and measuring means", shown for example in FIGs. 7D - 7M) and a septum gripper 52 configured for coupling implant 50 by gripping the atrial septum from both its sides (one side facing the right atrium and opposite side facing the left atrium). Implant body 51 has a body wall 53 enclosing an inner lumen 54 (shown in FIGs. 6B - 6C) sized for housing the sensing or/and measuring means, and may have a tubular structure, or any other structure suitable for housing the sensing or/and measuring mean(s).

Implant body 51 of implant 50 includes a first elongated wall segment 55 and a second elongated wall segment 56 (shown in FIG. 6C). Optionally, Implant body 51 includes a third elongated wall segment 57. According to some embodiments, third elongated wall segment 57 is configured for housing sensing or/and measuring means, although either or both first and third elongated wall segments, 55 and 57, may be used to house sensors / transducers.

The implant body 51 has an outer diameter within the range of about 1 mm to about 5 mm, optionally about 2 mm to about 5 mm, optionally about 2.5 mm to about 5 mm, or optionally about 3.5 mm to about 5 mm. According to some embodiments, the implant has a length within the range of about 8 mm and about 51 mm, optionally about 10 mm and about 51 mm.

Implant body 51 may be made or fabricated from a metal and may optionally include ceramic materials. Suitable materials from which implant body 50 is made of are those that provide biocompatibility and structural strength and allow effective radio frequency (RF) communication between the implant and an external unit to thereby facilitate harmonization of the data transmittal and processing. The metals suitable for manufacturing implant body 51 include, without limitation, metal alloys that include, for example titanium (hereinafter "Ti alloy"). The alloy may include, for example, Ti "grade II" or Titanium Grade I. Non-metallic materials suitable for manufacturing implant body 51 may include, without limitation, ceramics. Exemplary suitable ceramics may include zirconia, titania, or alumina.

According to some embodiments, first wall segment 55 or/and third wall segment 57 is formed of metal alloy. According to some embodiments, the second wall segment 56 is formed of a ceramic material such as zirconia or alumina. According to some embodiments, the second wall segment 56 houses an antenna, optionally in a form of a metal coil (as shown in FIG. 7F, for example), which is configured for transmitting or/and receiving radio-frequency electromagnetic waves.

Septum gripper 52 includes a flexible gripping sleeve 58 shapeable from an extended (slim) delivery form sized to pass through a septum opening in an atrial septum, to a non-bounded gripping form configured for gripping onto the atrial septum (shown in FIGs. 6A -6D). The gripping sleeve 58, when in the gripping form may have a structure that resembles double parallel flat plates that are facing each other.

According to some embodiments, the gripping sleeve 58 includes a proximal foldable unit 58a and a distal foldable unit 58b. Proximal foldable unit 58a and distal foldable unit 58b may each have a different shape or may both be structurally similar. Optionally, additionally or alternatively, proximal foldable unit 58a and distal foldable unit 58b may each have different dimensions (e.g. diameter or/and thickness) or may have similar or identical dimensions. Proximal foldable unit 58a and distal foldable unit 58b are each interconnected with and foldable relative to a spacing unit 59 that spaces apart proximal foldable unit 58a from distal foldable unit 58b.

As will be explained in more details below, spacing unit 59 is configured to accommodate the atrial septum and to allow gripping thereof by the foldable units 58a and 58b. Spacing unit 59, optionally together with at least portions of foldable units 58a and 58b closest thereto, are therefore adjustable or/and conformable and accommodative relative to a particular thickness (variable or relatively constant) of the atrial septum, which may vary between patients. Spacing unit 59 itself, when in a relaxed non-stresses form, may have a length equal to or smaller than a maximal thickness of the atrial septum in order to maintain a continuous compression force upon gripping an atrial septum.

According to some embodiments, the gripping sleeve 58 includes at least one of a non-foldable sleeve portion 60. The non-foldable sleeve portion may be non-shapeable. As used herein the term "non-shapeable" relates to a portion that is not changing in dimensions during sleeve change in shape when the septum gripper 52 transforms from a narrowed or/and stretched (delivery) form to a radially expanded (gripping) form. Non-foldable sleeve portion 60 may be disposed proximally to the proximal foldable unit 58a, distally to the distal foldable unit 58b, or both.

According to some embodiments, non-foldable sleeve portion 60 is about 2.5 mm or less, 2.2 mm or less, 2 mm or less, 1.8 mm or less, or 1.5 mm or less in inner dimension. According to some embodiments, inner dimensions may be inner diameter.

Gripping sleeve 58 may be made or fabricated from an elastic material capable of deforming from one configuration to another configuration. For example, gripping sleeve 58 may be made from a material having super elastic properties. Further, gripping sleeve 58 may be made from or may have shape memory properties (metal or polymer), or include locking mechanism for locking it in the second, deployed (gripping), configuration. According to some embodiments, gripping sleeve is made from a metal alloy. Suitable metal alloys include, but are not limited to nitinol (Ni-Ti).

In accordance with those embodiments, gripping sleeve 58 may be formed by pressing griping sleeve 58 over a rigid mandrel shaped and dimensioned substantially similarly with the in inner dimensions of gripping sleeve 58 when in the gripping form.

According to some embodiments, gripping sleeve 58 is made from a material that does not produce an adverse effect or that is compatible with human body (such as biocompatible). According to one embodiment, the gripping sleeve 58 is made or fabricated from a mesh. According to some embodiments, the mesh is formed by braiding of at least one wire. According to some embodiments, the number of wires that form the mesh is in between the range of 56 wires to 60 wires, or in between 35 wires to 45 wires.

According to some embodiments, at least one wire has a wire thickness of at least 80 µm, at least 100 µm or at least 150 µm. The wires may be round shaped in order to minimize damage to tissue, the implant or the delivery system. The wires may be braided at a ratio of 1:1 clock wise to counter clockwise.

According to some embodiments, at least a portion of septum gripper 52 (such as gripping sleeve 58, or the mesh or wire forming the sleeve 58) or/and the non-foldable portion(s) 60, or/and any connection means thereof, is encapsulated with electrically non-conductive layer. Optionally and alternatively, septum gripper 52 is only partially encapsulated. Encapsulation with a non-conductive layer(s) is utilized to electrically isolate the septum gripper 52 from the metal portions of implant body 51 and to thereby prevent data transmittal disturbances or interference. Suitable materials for encapsulations include polymers (such as Parylene C), and ceramics, glass, and oxides. According to some embodiments, the electrically non-conductive layer separates or/and spaces between body wall 53 or/and adjacent bodily tissue and blood and the gripping sleeve 58. Encapsulation results with a layer coating a material and may vary in thickness. The encapsulation layer thickness may range from several nanometers to several micrometers. An exemplary encapsulation thickness may be within the range of about 50 nm about 50 µm.

Optionally, additionally or alternatively, the electrically non-conductive layer may include an oxidative layer covering the material (e.g., natively oxidized nitinol).

As shown in FIG. 6C, implant body 51 of implant 50 includes a first elongated wall segment 55 of a first outer dimension and a second elongated wall segment 56 of a second outer dimension. Optionally, implant body 51 includes a third elongated wall segment 57 that may have an outer diameter greater than outer diameter of second elongated wall segment 56 or/and equals to the outer diameter of the first elongated wall segment 55. According to some embodiments, the outer dimension of the second elongated wall segment 56 is smaller than the outer dimension of the first elongated wall segment 55. In accordance with those embodiments, second elongated wall segment 56 allows to accommodate in a fixator and continuous manner, that prevents axial movement, non-foldable sleeve portion 60 including spacing unit 59.

Reference is now made to FIGs. 7A - 7M, which show different views of implant 100 and of sub-systems and components thereof. Implant 100 includes an implant body 101 having a tubular body wall 102 (shown in FIG. 7D, for example) and connected body segments.

In some embodiments, implant 100 is configured for coupling onto a chamber wall in a subject body, and configured for inductive coupling with an external unit (such as external unit 11 of FIG. 1B) positionable outside the subject body, and for transmitting digital signals to the external unit. Body wall 102 includes an electrically non-conductive wall section housing an RF antenna configured for facilitating the inductive coupling or/and the transmitting of the digital signals. Optionally, this non-conductive wall section is made from an electrically non-conductive material, and is optionally connected to a distal section or/and to a proximal section made from an electrically conductive material.

Body wall 102 encloses an inner lumen 103 (shown in FIG. 7E, for example) and configured for housing an RF antenna 104, an electronic circuit unit 105 or/and sensing or/and measuring means 106 (shown in FIG. 7F, for example), which are configured for sensing (detecting) or/and measuring, and optionally, monitoring, at least one physiological condition or/and parameter in a chamber of the heart.

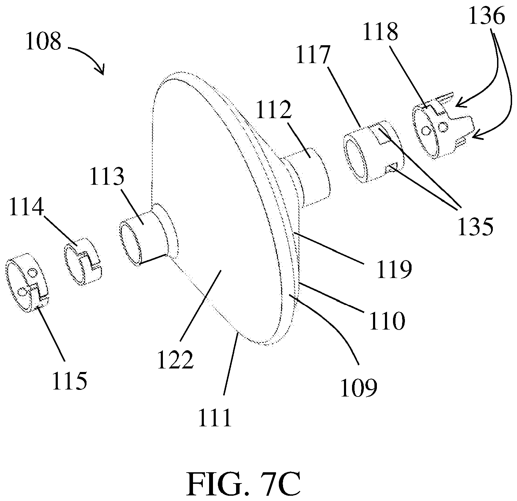

Implant 100 further includes a septum gripper 108 (shown, separately, in FIGs. 7B and 7C) including a flexible gripping sleeve 109. Flexible gripping sleeve 109 is fixedly connected to implant body 101 with a distal inner ring and slidably connected to the implant body with an axially slidable proximal inner ring. Flexible gripping sleeve 109 is selectively shapeable from a narrowed and stretched (delivery) form, when the proximal inner ring is withdrawn proximally away from the distal inner ring, to a radially expanded (gripping) form encompassing the non-conductive wall section, configured for gripping onto the chamber wall, when the proximal inner ring is advanced distally towards the distal inner ring. Flexible gripping sleeve 109 may include an electrically conductive mesh covered with an electrically insulating layer, and each of the proximal and distal inner rings is made from an electrically non-conductive material.

Gripping sleeve 109 includes a bellows-like structure, with a plurality of the foldable units, namely, a proximal foldable unit 110 and distal foldable unit 111. Septum gripper 108 further includes a proximal non-foldable sleeve portion 112 disposed proximally to the proximal foldable unit 110, and a distal non-foldable sleeve portion 113 disposed distally to the distal foldable unit 111 (shown in FIG. 7C, for example).

Distal non-foldable sleeve portion 113 is sandwiched between a sleeve fixation inner ring 114 and a sleeve fixation outer ring 115. According to some embodiments, sleeve fixation inner ring 114 is sized and shaped to mate or/and lock in a recess 116 (shown in FIG. 7E) in implant body 101, which forms a sleeve fixation area and prevents axial and rotation movement of distal non-foldable sleeve portion 113 during transformation (reshaping or/and motion) of septum gripper 108.

Proximal non-foldable sleeve portion 112 is sandwiched between a sleeve deploying inner ring 117 and a sleeve deploying outer ring 118. As will be explained in more details below, sleeve deploying inner ring 117 and sleeve deploying outer ring 118, and proximal non-foldable sleeve portion 112 sandwiched therebetween, are configured to selectively slide axially along implant body wall 102, as part of implantation of implant 100.

Septum gripper 108 or at least a portion or a part thereof (e.g., sleeve fixation inner ring 114, sleeve fixation outer ring 115, sleeve deploying inner ring 117, sleeve deploying outer ring 118, or/and flexible gripping sleeve 109) is made from a non-conductive material (e.g., plastic), or encapsulated with an electrically non-conductive layer, in order to minimize RF transmittal disturbances or interferences, between RF antenna 104 and a remote receiver/transmitter (such of external unit 11 of FIG. 1B, for example). Encapsulation with a non-conductive layer(s) can be utilized to electrically isolate the septum gripper 108 from direct contact with metal portions of implant body 101 and to thereby prevent data transmittal disturbances. Suitable materials for encapsulations may include polymers (e.g., Parylene C), and ceramics. Encapsulation may include an oxide layer. According to some embodiments, the electrically non-conductive layer or/and possible non-conductive spacers, separates or/and spaces between the body wall 102, or/and adjacent bodily tissue and blood, and the gripping sleeve 109.

Gripping sleeve 109 is configured such that in the gripping form, the proximal foldable unit 110 forms a proximal wing extending outwardly-radially relative to implant body 101 so as to form a first proximal surface 119 and a second proximal surface 120 (as shown in FIG. 8E) interconnected with a proximal edge 121. Similarly, gripping sleeve 109 is configured such that in the gripping form, distal foldable unit 111 forms a distal wing extending outwardly-radially relative to implant body 101 so as to form a first distal surface 122 and a second distal surface 123 (as shown in FIG. 8E) interconnected with a distal edge 124.

Gripping sleeve 109 is further configured such that the plurality of foldable units 110 and 111 are configured for gripping by pressing the second proximal surface 120 or/and the proximal edge 121 against a proximal side of an atrial septum and pressing the second distal surface 123 or/and the distal edge 124 against a distal side of the atrial septum, under a continuous compression force greater than a predetermined minimal force value. As used herein the term "continuous compression force" relates to a continuous compression force applied by the foldable units 110 and 111 of the gripping sleeve 109 on the atrial septum. According to some embodiments, the gripping sleeve 109 is configured such to continuously for long periods, for example a year or so compress the atrial septum. The continuous compression force is greater than a predetermined minimal force taken, for example, from between about 0.1 gr and about 100 gr, optionally particularly between about 0.5 gr and about 50 gr, optionally particularly from about 1 gr and about 10 gr.

According to some embodiments, the bellows-like structure of foldable units 110 and 111 is configured with a coefficient of static friction chosen so as to prevent relative motion of the septum gripper 108 relative to the atrial septum under normal stresses (i.e., physiological stresses) applied thereto in the chamber of the heart.

According to some embodiments, when gripping sleeve 109 is in the gripping form, the first proximal surface 119 and the second proximal surface 120 are formed as nested conic structures extending proximally away from the proximal edge 121 separated by the proximal edge 121. According to some embodiments, when gripping sleeve 109 is in the gripping form, the first distal surface 122 and the second distal surface 123 are formed as nested conic structures extending distally away from the distal edge 124 separated by the distal edge 124.

Sensing or/and measuring means 106 is encapsulated in a sensory cup 125, and is electrically connected to electronic circuit unit 105 and to RF antenna 104.

Sensing or/and measuring means 106 and sensory cup 125 are provided in this exemplary embodiment at distal end of implant 100 (and configured for positioning in the left atrium) when gripped to the atrial septum, but may be positioned on the proximal and distal end of the implant or that the implant will include two such units (or more), provided at both ends thereof, for example. The electronic circuit unit 105 includes dedicated application-specific integrated circuit (ASIC) mounted on a miniature printed circuit board (PCB) applied with further discreet elements. The RF antenna 104 is electrically connected to the PCB. The RF antenna 104 may, for example, include a core (e.g., metallic) wrapped with a conductive coil. The PCB and the antenna 104 may be electrically connected via soldering or via any other relevant method. The sensing or/and measuring means 106 may further include two non-(electrically) conductive spacers 126 and 127 (shown in FIGs. 7G - 7H). The spacers 126 and 127 may be made of either a polymer or/and an inorganic compound and are configured for fixating the means in a specific plan within the implant body 101. According to some embodiments, the sensing or/and measuring means 106 are confined within and may be integrated into implant body 101.

Sensing or/and measuring means 106, within sensory cup 125, may include a membrane 128, a ring 129 and a ceramic assembly 130, which may, for example, connect to or/and store pressure traducers 131. Sensory cup 125 may be formed by metal or non-metal material, or/and be encapsulated, covered or have lining with a polymer. For example, a thin polymeric layer may be added to inner surfaces of the sensory cup 125, as well as, possibly, an outside coating, generating an outer thin encapsulation that surrounds the entire cup perimeter. Coating may use medical grade silicone rubber, e.g. MED4-4420

Sensory cup 125 includes a plurality (e.g., three) of feed through pins 132 which may be brazed within pre-shaped ceramic assembly 130. According to some embodiments, a single feed through pin 132 is connected to two pressure traducers 131 at a first pole while two other feed through pins 132 are each connected to one of these two pressure transducers 131 at their second pole. A fourth feed through pin 133 may be non-connected to a pressure transducer and applied for ground. Each feed through pin 132 and 133 may have a diameter within the range of about 0.1 mm and bout 0.5 mm, for example 0.3 mm. Sensory cup 125 may be made of a metallic material, including, without limitation Titanium II. Sensory cup 125 may be from about 1.5 mm and about 5 mm in diameter, for example about 2.5 mm.

Reference is now made to FIGs. 8A - 8G which show use of an implant deployment device 200 for the delivery and deployment of implant 100, releasably connected thereto. Implant deployment device 200 optionally includes at least one of a sheath 201, a fluid inlet 202 communicating with interior of sheath 201, an implant delivery loader 203, and a rod or a tether 204. Implant deployment device 200 may further include clip-deploying arm 205, configured for releasably coupling with septum gripper 108 and thereby applicable for shifting septum gripper 108 between the delivery form and the gripping form by unhindered sliding along a segment of body wall 102 of implant 100 or/and of outer periphery of sheath 201. Optionally the clip-deploying arm 205 has a smooth surface. Implant deployment device 200, via tether 204, is attached to the proximal end of implant body 101, and can be used for maintaining the implant body 201 in place when clip-deploying arm 205 is put to motion in order to achieve relative motion between the septum gripper 108 and implant body 101.

Rod 204 is connectable to implant body 101 optionally with a threading component 207 which is threadable into a mating threading part 137 connected to or is part of proximal end of implant body 101. When connected to implant 100 with both rod/tether 204 and clip-deploying arm 205, the implant deployment device 200 can be used for deploying and fixating implant 100 within the heart septum. Clip-deploying arm 205 may then be applied for grasping the septum wall using septum gripper 108. The clip-deploying arm 205 may then be released from septum gripper 108, prior to, followed by or in parallel to disconnecting tether 204 from proximal end of implant body 101, once the implant has been positioned.

Clip-deploying arm 205 is releasably coupled to implant 100 at recesses 135 provided on sleeve deploying inner ring 117 of septum gripper 108. In some embodiments, sleeve deploying inner ring 117, when coupled with clip-deploying arm 205 and during deployment of septum gripper 108, is configured as a sliding element, optionally having a smooth surface in contact with implant body 101. In some embodiments, sleeve deploying inner ring 117 is also made from a non-conductive material.

As detailed above, a free end of gripping sleeve 109 is sandwiched with interconnecting sleeve deploying inner ring 117 and sleeve deploying outer ring 118, wherein the sleeve deploying outer ring 118 is crown-like shaped with proximally pointing concavities 136 for uncovering recesses 135 (when connected thereto above) for facilitating coupling of clip-deploying arm 205.

According to some embodiments, and as will be explained below, gripping sleeve 109 is fixedly extendable or/and compressible to a chosen length or/and shape in between more than two distinct forms, including a delivery form and a gripping form, when coupled with a distal end of the implant deployment device 200. According to some embodiments, septum gripper 108 coupling to the delivery system 200 enables full control on the deployment of the gripper 108. Such control affords selective positioning of the foldable units 110 and 111, separately, while their design optionally allows some degree of conformity in response to local anatomy or/and external stresses (e.g., applied by anatomy of the heart).

The procedure of implanting implant 100 within the atrial septum includes puncture and forming of a septum opening using any standard trans septal puncturing kit and in according to the normal procedure. The puncturing is associated with routing and inserting, for example, a lead including a punctuator through the septum for effecting opening within the septum. Implant deployment device 200 connected to implant 100 is then routed through the septum opening. When in the delivery configuration, gripping sleeve 109 of septum gripper 108 is extended to be sized for passing through the septum opening. Gripping sleeve 109 is thus forced to stretch or/and confined with an outer boundary having an outer boundary inner diameter. As the implant is inserted to protrude into the left atrium, when in the delivery configuration, the distal foldable unit 111 is first to be deployed (FIG. 8F). Following stabilization of the distal foldable unit 111 against the left atrial septum wall, the deployment device is stabilized to facilitate deployment of the proximal foldable unit 110 against the right atrial septum wall. Consequently, shifting from the delivery configuration to the gripping configuration (i.e., a non-bounded preformed gripping form) includes gripping of the griping sleeve 109 onto the atrial septum.

The implant body 101 is further fixated to or/and stabilized within the septal walls in a chosen orientation. As used herein the term "chosen orientation" refers to the orientation of implant 100 that the physician chooses for gripping the atrial septum by the gripping sleeve 109. Chosen orientation is a tridimensional orientation relative to a center point of the septum opening and includes implant body extending through the septum opening such that an operational member thereof (i.e., sensing or/and measuring means) is provided in the target chamber of the heart.

The implant 100 may be retrieved, if and when necessary in a manner similar to the deployment of the implant with the exception that the foldable units 110 and 111 are unfolded. Specifically, the proximal foldable unit 110 is firstly unfolded and thereafter the distal foldable unit 111 is unfolded to thereby afford retrieval and removal of the implant from the body.

It is to be fully understood that certain aspects, characteristics, and features, of the invention, which are, for clarity, illustratively described and presented in the context or format of a plurality of separate embodiments, may also be illustratively described and presented in any suitable combination or subcombination in the context or format of a single embodiment. Conversely, various aspects, characteristics, and features, of the invention which are illustratively described and presented in combination or sub combination in the context or format of a single embodiment, may also be illustratively described and presented in the context or format of a plurality of separate embodiments.

Although the invention has been described in conjunction with specific embodiments thereof, it is evident that many alternatives, modifications, and variations will be apparent to those skilled in the art. Accordingly, it is intended to embrace all such alternatives, modifications, and variations that fall within the scope of the appended claims.

## Claims

1. An implant (100) for sensing or/and measuring a physiological parameter in a subject's body organ separated by an organ wall, the implant comprising:
an implant body (101) comprising a rigid body wall (102, 125, 137) enclosing an inner lumen (103) sized and configured for housing physiological parameter sensing or/and measuring means (106) and an antenna (104) configured for transmitting or/and receiving radio-frequency electromagnetic waves, said body wall (102, 125, 137) including coupling means (137) at a proximal end thereof and configured for coupling with a distal end (207) of an implant deployment device (200); and
a septum gripper (108) comprising a flexible gripping sleeve (109) comprising an electrically conductive material encapsulated by an electrically non-conductive layer, said gripping sleeve (109) being configured and selectively shapeable from a radially narrowed and axially stretched form, sized to pass through a septum opening in the organ wall, to a radially expanded form, suitable for gripping onto the organ wall from opposite sides thereof and around said septum opening, such that said implant body (101) is fixatable or/and stabilizable across the organ wall and through said septum opening, so as to facilitate the sensing or/and measuring in the subject's body organ,
wherein:
(i) a distal end (113) of the gripping sleeve (109) is fixedly connected to a periphery of said rigid body wall (102, 125, 137) at a sleeve fixation area (116) between a center point and a distal end of said body wall (102, 125, 137);
(ii) a proximal end of the septum gripper (108) comprises a sliding element (117) made from a non-conductive material, configured to slide axially relative to the implant body, so that the gripping sleeve (109) transitions selectively between the radially narrowed and axially stretched form and the radially expanded form; and
(iii) said rigid body wall (102, 125, 137) includes an electrically non-conductive wall section (102) housing the antenna.

2. The implant according to claim 1, wherein the flexible gripping sleeve (109) comprises an electrically conductive mesh.

3. The implant according to claim 1 or 2, said gripping sleeve includes a bellows-like structure comprising a plurality of foldable units, including a proximal foldable unit (110) and a distal foldable unit (111), wherein, when said gripping sleeve is in said expanded form,
said proximal foldable unit (110) forms a proximal wing extending outwardly-radially relative to said implant body, so as to form a first proximal surface (119) and a second proximal surface (120) interconnected with a proximal edge (121), and
said distal foldable unit (111) forms a distal wing extending outwardly-radially relative to said implant body, so as to form a first distal surface (122) and a second distal surface (123) interconnected with a distal edge (124).

4. The implant according to claim 3, wherein said bellows-like structure is configured for gripping the organ wall, by pressing said second proximal surface or/and said proximal edge against a proximal side of the organ wall and pressing said second distal surface or/and said distal edge against a distal side of the organ wall, under a continuous compression force greater than a predetermined minimal force value.

5. The implant according to claim 3, wherein said proximal foldable unit and said distal foldable unit are spaced or/and interconnected with, and foldable relative to, a spacing unit.

6. The implant according to claim 4 or 5, wherein, when said gripping sleeve (109) is in said expanded form,
said first proximal surface and said second proximal surface are formed as nested conic structures extending proximally away from said proximal edge, or formed as parallel flat structures, separated by said proximal edge; or/and
said first distal surface and said second distal surface are formed as nested conic structures extending distally away from said distal edge, or formed as parallel flat structures, separated by said distal edge.

7. The implant according to any of the preceding claims, wherein said septum gripper (108) comprises a non-foldable sleeve portion (113) provided at a distal end of the flexible gripping sleeve (109).

8. The implant according to any of the preceding claims, wherein said gripping sleeve (109) is formed by pressing over a rigid mandrel shaped and dimensioned with inner dimensions of said gripping sleeve in said expanded form.

9. The implant according to any of the preceding claims, wherein said septum gripper comprises a sleeve deploying inner ring comprising or being configured as said sliding element (117).

10. The implant according to claim 9, wherein a proximal end (112) of said gripping sleeve is sandwiched between said sleeve deploying inner ring (117) and a sleeve deploying outer ring (118), wherein said sleeve deploying outer ring (118) is configured for coupling with a sleeve deploying arm (205) provided with said implant deployment device.

11. The implant according to any of the preceding claims, wherein said body wall includes a first elongated wall segment of a first outer dimension and a second elongated wall segment (102) of a second outer dimension smaller than said first outer dimension, wherein said gripping sleeve (109) is configured to cover said first and second wall segments when in said narrowed form.

12. The implant according to claim 11, comprising a third wall segment (125) distally to said second wall segment, configured for housing electronic or/and electro-mechanical components.

13. The implant according to any of the preceding claims, wherein the septum gripper (108) further comprises a distal outer ring (115) and a non-conductive distal inner ring (114), which sandwich the distal end (113) of the gripping sleeve (109).

14. The implant according to any of claims 1-8, wherein said flexible gripping sleeve (109) is fixedly connected to the implant body with a distal inner ring (114) and slidably connected to said implant body with an axially slidable proximal sleeve deploying inner ring, wherein said gripping sleeve is in said radially narrowed and axially stretched form, when the proximal sleeve deploying inner ring is withdrawn proximally away from the distal inner ring, to said radially expanded form, when said proximal sleeve deploying inner ring is advanced distally towards the distal inner ring.

## Patentansprüche

1. Ein Implantat (100) zum Erfassen oder/und Messen eines physiologischen Parameters in einem Körperorgan einer Person, das durch eine Organwand getrennt ist, wobei das Implantat umfasst: einen Implantatkörper (101), der eine starre Körperwand (102, 125, 137) umfasst, die ein inneres Lumen (103) umschließt, das so bemessen und konfiguriert ist, dass es Mittel (106) zum Erfassen oder/und Messen eines physiologischen Parameters und eine Antenne (104) aufnimmt, die so konfiguriert ist, dass sie elektromagnetische Hochfrequenzwellen sendet oder/und empfängt, wobei die Körperwand (102, 125, 137) an ihrem proximalen Ende Kopplungsmittel (137) einschließt, die so konfiguriert sind, dass sie mit einem distalen Ende (207) einer Implantatentfaltungsvorrichtung (200) gekoppelt werden können; und einen Septumgreifer (108), der eine flexible Greifhülse (109) umfasst, die ein elektrisch leitendes Material umfasst, das von einer elektrisch nicht leitenden Schicht eingekapselt ist, wobei die Greifhülse (109) so konfiguriert und selektiv formbar ist, dass sie von einer radial verengten und axial gestreckten Form, die so bemessen ist, dass sie durch eine Septumöffnung in der Organwand passt, in eine radial expandierte Form, die zum Greifen an der Organwand von gegenüberliegenden Seiten davon und um die Septumöffnung herum geeignet ist, so dass der Implantatkörper (101) quer über die Organwand und durch die Septumöffnung hindurch fixierbar oder/und stabilisierbar ist, um das Erfassen oder/und Messen in dem Körperorgan der Person zu erleichtern, wobei:
(i) ein distales Ende (H3) der Greifhülse (109) fest mit einer Peripherie der starren Körperwand (102, 125, 137) an einem Hülsenfixierungsbereich (H6) zwischen einem Mittelpunkt und einem distalen Ende der Körperwand (102, 125, 137) verbunden ist;
(ii) ein proximales Ende des Septumgreifers (108) ein Gleitelement (117) aus einem nichtleitenden Material umfasst, das so konfiguriert ist, dass es relativ zum Implantatkörper axial gleitet, so dass die Greifhülse (109) selektiv zwischen der radial verengten und axial gestreckten Form und der radial expandierten Form übergeht; und
(iii) die starre Körperwand (102, 125, 137) einen elektrisch nichtleitenden Wandabschnitt (102) einschließt, der die Antenne aufnimmt.

2. Implantat nach Anspruch 1, wobei die flexible Greifhülse (109) ein elektrisch leitfähiges Netz umfasst.

3. Implantat nach Anspruch 1 oder 2, wobei die Greifhülse eine faltenbalgartige Struktur einschließt, die eine Vielzahl von faltbaren Einheiten umfasst, einschließlich einer proximalen faltbaren Einheit (110) und einer distalen faltbaren Einheit (111), wobei die proximale faltbare Einheit (110), wenn sich die Greifhülse in der expandierten Form befindet, einen proximalen Flügel bildet, der sich relativ zu dem Implantatkörper radial nach außen erstreckt, so dass eine erste proximale Oberfläche (119) und eine zweite proximale Oberfläche (120) gebildet werden, die mit einer proximalen Kante (121) zwischenverbunden sind, und die distale faltbare Einheit (111) einen distalen Flügel bildet, der sich relativ zu dem Implantatkörper radial nach außen erstreckt, so dass eine erste distale Oberfläche (122) und eine zweite distale Oberfläche (123) gebildet werden, die mit einer distalen Kante (124) zwischenverbunden sind.

4. Implantat nach Anspruch 3, wobei die faltenbalgartige Struktur so konfiguriert ist, dass sie die Organwand ergreift, indem sie die zweite proximale Oberfläche oder/und die proximale Kante gegen eine proximale Seite der Organwand drückt und die zweite distale Oberfläche oder/und die distale Kante gegen eine distale Seite der Organwand drückt, und zwar unter einer kontinuierlichen Druckkraft, die größer ist als ein vorbestimmter minimaler Kraftwert.

5. Implantat nach Anspruch 3, wobei die proximale faltbare Einheit und die distale faltbare Einheit beabstandet oder/und mit einer Beabstandungseinheit zwischenverbunden und relativ zu dieser faltbar sind.

6. Implantat nach Anspruch 4 oder 5, wobei, wenn die Greifhülse (109) in der expandierten Form ist, die erste proximale Oberfläche und die zweite proximale Oberfläche als verschachtelte konische Strukturen gebildet sind, die sich proximal von der proximalen Kante weg erstrecken, oder als parallele flache Strukturen gebildet sind, die durch die proximale Kante getrennt sind; oder/und die erste distale Oberfläche und die zweite distale Oberfläche als verschachtelte konische Strukturen gebildet sind, die sich distal von der distalen Kante weg erstrecken, oder als parallele flache Strukturen gebildet sind, die durch die distale Kante getrennt sind.

7. Implantat nach einem der vorhergehenden Ansprüche, wobei der Septumgreifer (108) einen nicht faltbaren Hülsenabschnitt (113) umfasst, der an einem distalen Ende der flexiblen Greifhülse (109) bereitgestellt ist.

8. Implantat nach einem der vorhergehenden Ansprüche, wobei die Greifhülse (109) durch Pressen über einen starren Dorn geformt wird, der mit den Innenabmessungen der Greifhülse in der expandierten Form geformt und dimensioniert ist.

9. Implantat nach einem der vorhergehenden Ansprüche, wobei der Septumgreifer einen hülsenentfaltenden Innenring umfasst, der das Gleitelement (117) umfasst oder als solches gebildet ist.

10. Implantat nach Anspruch 9, wobei ein proximales Ende (112) der Greifhülse zwischen dem hülsenentfaltenden Innenring (117) und einem hülsenentfaltenden Außenring (118) angeordnet ist, wobei der hülsenentfaltende Außenring (118) zum Koppeln mit einem hülsenentfaltenden Arm (205) konfiguriert ist, der mit der Implantatentfaltungsvorrichtung bereitgestellt ist.

11. Implantat nach einem der vorhergehenden Ansprüche, wobei die Körperwand ein erstes längliches Wandsegment mit einer ersten Außenabmessung und ein zweites längliches Wandsegment (102) mit einer zweiten Außenabmessung, die kleiner als die erste Außenabmessung ist, einschließt, wobei die Greifhülse (109) so konfiguriert ist, dass sie das erste und das zweite Wandsegment bedeckt, wenn sie sich in der verengten Form befindet.

12. Implantat nach Anspruch 11, umfassend ein drittes Wandsegment (125) distal zu dem zweiten Wandsegment, das zum Aufnehmen von elektronischen oder/und elektromechanischen Komponenten konfiguriert ist.

13. Implantat nach einem der vorhergehenden Ansprüche, wobei der Septumgreifer (108) ferner einen distalen Außenring (115) und einen nicht leitenden distalen Innenring (114) umfasst, die das distale Ende (113) der Greifhülse (109) umschließen.

14. Implantat nach einem der Ansprüche 1-8, wobei die flexible Greifhülse (109) mit einem distalen Innenring (114) fest mit dem Implantatkörper verbunden ist und mit einem axial verschiebbaren proximalen Hülsenentfaltungsinnenring gleitend mit dem Implantatkörper verbunden ist, wobei sich die Greifhülse in der radial verengten und axial gestreckten Form befindet, wenn der proximale Hülsenentfaltungsinnenring proximal vom distalen Innenring weggezogen wird, und in die radial expandierte Form übergeht, wenn der proximale Hülsenentfaltungsinnenring distal zum distalen Innenring hin vorgeschoben wird.

## Revendications

1. Implant (100) pour détecter et/ou mesurer un paramètre physiologique dans un organe corporel d'un sujet séparé par une paroi d'organe, l'implant comprenant :
un corps d'implant (101) comprenant une paroi corporelle (102, 125, 137) rigide entourant une lumière interne (103) dimensionnée et configurée pour loger des moyens (106) de détection et/ou de mesure de paramètres physiologiques et une antenne (104) configurée pour émettre et/ou recevoir des ondes électromagnétiques radiofréquence, ladite paroi corporelle (102, 125, 137) comprenant des moyens d'accouplement (137) au niveau d'une extrémité proximale de celle-ci et configurée pour être accouplée à une extrémité distale (207) d'un dispositif (200) de déploiement d'implant ; et
un organe (108) de préhension de septum comprenant un manchon de préhension (109) flexible comprenant un matériau électroconducteur encapsulé par une couche non électroconductrice, ledit manchon de préhension (109) étant configuré et pouvant être sélectivement mis en forme à partir d'une forme radialement rétrécie et axialement étirée, dimensionnée pour passer à travers une ouverture de septum dans la paroi d'organe, en une forme radialement étendue, appropriée pour s'agripper à la paroi d'organe depuis les côtés opposés de celle-ci et autour de ladite ouverture de septum, de sorte que ledit corps d'implant (101) peut être fixé et/et stabilisé à travers la paroi d'organe et à travers ladite ouverture de septum, de manière à faciliter la détection et/ou la mesure dans l'organe corporel du sujet,
dans lequel :
(i) une extrémité distale (113) du manchon de préhension (109) est reliée de manière fixe à une périphérie de ladite paroi corporelle (102, 125, 137) rigide au niveau d'une zone (116) de fixation de manchon entre un point central et une extrémité distale de ladite paroi corporelle (102, 125, 137) ;
(ii) une extrémité proximale de l'organe (108) de préhension de septum comprend un élément coulissant (117) réalisé en un matériau non conducteur, configuré pour coulisser axialement par rapport au corps d'implant, de sorte que le manchon de préhension (109) effectue sélectivement une transition entre la forme radialement rétrécie et axialement étirée et la forme axialement étendue ; et
(iii) ladite paroi corporelle (102 125, 137) comprend une section de paroi (102) non électroconductrice logeant l'antenne.

2. Implant selon la revendication 1, dans lequel ledit manchon de préhension (109) flexible comprend un maillage électroconducteur.

3. Implant selon la revendication 1 ou 2, ledit manchon de préhension comprend une structure de type soufflet comprenant une pluralité d'unités pliables, comprenant une unité pliable proximale (110) et une unité pliable distale (111), dans lequel, lorsque ledit manchon de préhension est dans ladite forme étendue, ladite unité pliable proximale (110) forme une aile proximale s'étendant radialement vers l'extérieur par rapport audit corps d'implant, de manière à former une première surface proximale (119) et une seconde surface proximale (120) interconnectées avec un bord proximal (121), et ladite unité pliable distale (111) forme une aile distale s'étendant radialement vers l'extérieur par rapport audit corps d'implant, de manière à former une première surface distale (122) et une seconde surface distale (123) interconnectées avec un bord distal (124).

4. Implant selon la revendication 3, dans lequel ladite structure de type soufflet est configurée pour saisir la paroi d'organe, en appuyant ladite seconde surface proximale ou/et ledit bord proximal contre un côté proximal de la paroi d'organe et en appuyant ladite seconde surface distale ou/et ledit bord distal contre un côté distal de la paroi d'organe, sous une force de compression continue supérieure à une valeur de force minimale prédéterminée.

5. Implant selon la revendication 3, dans lequel ladite unité pliable proximale et ladite unité pliable distale sont espacées et/ou interconnectées avec, et pliables par rapport à, une unité d'espacement.

6. Implant selon la revendication 4 ou 5, dans lequel, lorsque ledit manchon de préhension (109) est dans ladite forme étendue,
ladite première surface proximale et ladite seconde surface proximale sont formées en tant que structures coniques emboîtées s'étendant de manière proximale à l'écart dudit bord proximal, ou formées en tant que structures plates parallèles, séparées par ledit bord proximal ; et/ou
ladite première surface distale et ladite seconde surface distale sont formées en tant que structures coniques emboîtées s'étendant de manière distale à l'écart dudit bord distal, ou formées en tant que structures plates parallèles, séparées par ledit bord distal.

7. Implant selon l'une quelconque des revendications précédentes, dans lequel ledit organe (108) de préhension de septum comprend une partie (113) de manchon non pliable prévue à une extrémité distale du manchon de préhension (109) flexible.

8. Implant selon l'une quelconque des revendications précédentes, dans lequel ledit manchon de préhension (109) est formé par pressage sur un mandrin rigide mis en forme et dimensionné avec les dimensions intérieures dudit manchon de préhension dans ladite forme étendue.

9. Implant selon l'une quelconque des revendications précédentes, dans lequel ledit organe de préhension de septum comprend un anneau intérieur de déploiement de manchon comprenant ou étant configuré en tant que ledit élément coulissant (117).

10. Implant selon la revendication 9, dans lequel une extrémité proximale (112) dudit manchon de préhension est prise en sandwich entre l'anneau (117) intérieur de déploiement de manchon et un anneau (118) extérieur de déploiement de manchon, dans lequel ledit anneau (118) extérieur de déploiement de manchon est configuré pour s'accoupler avec un bras (205) de déploiement de manchon pourvu dudit dispositif de déploiement d'implant.

11. Implant selon l'une quelconque des revendications précédentes, dans lequel ladite paroi corporelle comprend un premier segment de paroi allongé d'une première dimension extérieure et un deuxième segment de paroi (102) allongé d'une seconde dimension extérieure inférieure à ladite première dimension extérieure, dans lequel ledit manchon de préhension (109) est configuré pour couvrir lesdits premier et deuxième segments de paroi lorsqu'il est dans ladite forme rétrécie.

12. Implant selon la revendication 11, comprenant un troisième segment de paroi (125) de manière distale audit deuxième segment de paroi, configuré pour loger des composants électroniques et/ou électromécaniques.

13. Implant selon l'une quelconque des revendications précédentes, dans lequel ledit organe (108) de préhension de septum comprend en outre un anneau (115) extérieur distal et un anneau (114) intérieur distal non conducteur, qui prennent en sandwich l'extrémité distale (113) du manchon de préhension (109).

14. Implant selon l'une quelconque des revendications 1 à 8, dans lequel ledit manchon de préhension (109) flexible est relié de manière fixe au corps d'implant avec l'anneau (114) intérieur distal et relié en coulissement audit corps d'implant avec un anneau intérieur de déploiement de manchon proximal pouvant coulisser axialement, dans lequel ledit manchon de préhension est dans ladite forme radialement rétrécie et axialement étirée, lorsque l'anneau intérieur de déploiement de manchon proximal est retiré de manière proximale à l'écart de l'anneau intérieur distal, en ladite forme axialement étendue, lorsque l'anneau intérieur de déploiement de manchon proximal est avancé de manière distale vers l'anneau intérieur distal.
